# EUROPEAN PATENT APPLICATION

(11) **EP 4 635 420 A1**
(43) Date of publication of application: **22.10.2025**
(21) Application number: 24170705.8
(22) Date of filing: 17.04.2024
(51) Int. Cl.: A61B 6/50

(54) **VIEWING ANGLE FOR ACQUIRING PROJECTION DATA**

(71) Applicant: Koninklijke Philips N.V., 5656 AG Eindhoven (NL)
(72) Inventor: WISSEL, Tobias, Eindhoven (NL); BUERGER, Christian, Eindhoven (NL); BAKKER, Bart Jacob, 5656AG Eindhoven (NL); OLIVAN BESCOS, Javier, Eindhoven (NL)
(74) Representative: Philips Intellectual Property & Standards

(57) **Abstract**

A system (100) for providing a viewing angle for acquiring projection data of a vascular region, is disclosed. The system includes one or more processors (110) configured to receive volumetric data (120) representing the vascular region (130). The one or more processors (110) are also configured to determine, using the volumetric data (120), and based on a simulated motion (140) of the vascular region (130) and/or based on a motion of the vascular region (130) represented within the volumetric data (120), a value of a viewing angle for acquiring projection data (150) representing the vascular region (130). The one or more processors (110) are also configured to output the value of the viewing angle.

## Description

### TECHNICAL FIELD

The present disclosure relates to providing a viewing angle for acquiring projection data of a vascular region. A system, a computer-implemented method, a computer program product, and a projection X-ray imaging system that includes the system, are disclosed.

### BACKGROUND

Vascular procedures are often performed using projection images. For instance, percutaneous vascular treatment procedures rely heavily on projection X-ray images. However, projection X-ray images lack depth information. This lack of depth information can give a misleading impression of the vasculature, particularly if a projection X-ray image is acquired using a sub-optimal viewing angle with respect to the anatomy. For instance, a projection X-ray image of a stenosis that is acquired with a sub-optimal viewing angle can give a misleading impression of the length, and also the diameter, of the stenosis. This can adversely affect the diagnosis, and also the treatment, of the vessel. For example, it can adversely affect the selection, and also the positioning, of a stent in the vessel.

In order to overcome such drawbacks, projection X-ray images are often acquired using one or more standard viewing angles with respect to the anatomy. For example, one of the standard viewing angles that is used for treating lesions in the Left Anterior Descending "LAD" cardiac artery, is at Left Anterior Oblique "LAO" 40 degrees, Cranial 20 degrees. Sometimes, physicians use a standard viewing angle as a starting point, and subsequently improve upon the standard viewing angle based on acquired projection X-ray images. However, determining the viewing angle in this manner is time-consuming. Another technique for determining a viewing angle for acquiring projection X-ray images involves the use of a 3D model of the patient's vasculature, as described in the document WO 2004/012152 A2.

However, there remains a need to improve the way in which viewing angles are determined for acquiring projection X-ray images of the vasculature.

### SUMMARY

According to one aspect of the present disclosure, a system for providing a viewing angle for acquiring projection data of a vascular region, is provided. The system includes one or more processors configured to:
receive volumetric data representing the vascular region;
determine, using the volumetric data, and based on a simulated motion of the vascular region and/or based on a motion of the vascular region represented within the volumetric data, a value of a viewing angle for acquiring projection data representing the vascular region; and
output the value of the viewing angle.

The inventors have observed that a drawback of known techniques for determining viewing angles for acquiring projection data of the vasculature is that they are optimized under the assumption that the shape, and also the position, of the vasculature is static. Such viewing angles may be optimal for acquiring projection data of the vasculature when the actual shape and position of the vasculature corresponds to the assumed, static, shape and position. However, in practice, the vasculature is typically subjected to various types of motion, and this motion changes the shape, and also the position, of the vasculature. The source of the motion may be cardiac motion, respiratory motion, bulk motion of the anatomy, and so forth. The failure to account for such motion gives rise to projection X-ray images that are sub-optimal. The resulting projection X-ray images may even be misleading due to vessel overlaps, and also increased amounts of vessel foreshortening. Since the system described above determines a viewing angle for acquiring projection data using volumetric data representing the vascular region based on a simulated motion of the vascular region, and/or based on a motion of the vascular region represented within the volumetric data, the system takes account of changes in the shape and position of the vasculature and consequently provides a more optimal value of the viewing angle. The use of the viewing angle provided by the system therefore reduces the risk of acquiring projection X-ray images that give a misleading impression of the vasculature.

Further aspects, features, and advantages of the present disclosure will become apparent from the following description of examples, which is made with reference to the accompanying drawings.

### BRIEF DESCRIPTION OF THE DRAWINGS

Fig. 1 is a schematic diagram illustrating an example of a system 100 for providing a viewing angle for acquiring projection data of a vascular region, in accordance with some aspects of the present disclosure.
Fig. 2 is a flowchart illustrating an example of a computer-implemented method of providing a viewing angle for acquiring projection data of a vascular region, in accordance with some aspects of the present disclosure.
Fig. 3 is a schematic diagram illustrating an example of the generation of a virtual projection 130^{VP} of a vascular region 130 from a viewing angle α₁, β₁, of a virtual projection X-ray imaging system 210^{V} with respect to a vascular region 130, in accordance with some aspects of the present disclosure.
Fig. 4 is a schematic diagram illustrating a first example of a viewing angle α of a projection X-ray imaging system 210 with respect to a vascular region 130, in accordance with some aspects of the present disclosure.
Fig. 5 is a schematic diagram illustrating a second example of a viewing angle β of a projection X-ray imaging system 210 with respect to a vascular region 130, in accordance with some aspects of the present disclosure.
Fig. 6 is a schematic diagram illustrating an example of the generation of virtual projections 130^{VP}(αᵢ) of a vascular region 130 from corresponding viewing angles αᵢ of a virtual projection X-ray imaging system 210^{V} with respect to a vascular region 130, in accordance with some aspects of the present disclosure.
Fig. 7 is a schematic diagram illustrating the effect of cardiac motion on vessel foreshortening, in accordance with some aspects of the present disclosure.
Fig. 8 is a schematic diagram illustrating an example of a graphical representation 170 of a vascular region 130 including an example of a viewing angle α₁, β₁, that has been optimized for minimum foreshortening around the LAD-D1 bifurcation in the ES cardiac phase, in accordance with some aspects of the present disclosure.
Fig. 9 is a schematic diagram illustrating an example of a graphical representation 170 of a vascular region 130 including an indication of the amount of foreshortening observed in the ED cardiac phase for a viewing angle α₁, β₁, that has been optimized for minimum foreshortening around the LAD-D1 bifurcation in the ES cardiac phase, in accordance with some aspects of the present disclosure.
Fig. 10 is a schematic diagram illustrating an example of various aspects of a method of providing a viewing angle α₁, β₁, for acquiring projection data of a vascular region, in accordance with some aspects of the present disclosure.

### DETAILED DESCRIPTION

Examples of the present disclosure are provided with reference to the following description and Figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a computer-implemented method, may be implemented in a computer program product, and in a system, in a corresponding manner.

In the following description, reference is made to examples of a system for providing a viewing angle for acquiring projection data of a vascular region. In some examples, the vascular region includes a portion of the cardiac vasculature. It is, however, to be appreciated that the portion of the cardiac vasculature serves only as an example of a vascular region. In general, the vascular region may be any vascular region within the anatomy. For instance, the vascular region may alternatively include a portion of the pulmonary vasculature, the peripheral vasculature, the vasculature of the brain, and so forth.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid-state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray^{™} and DVD.

It is also noted that some operations that are described as being performed by the one or more processors of the system disclosed herein may be implemented using artificial intelligence techniques. Suitable techniques may include machine learning techniques, deep learning techniques, and neural networks. For instance, one or more neural networks, may be trained in a supervised, or in some cases unsupervised, manner, to implement the operations that are performed by the one or more processors.

As mentioned above, there remains a need to improve the way in which viewing angles are determined for acquiring projection X-ray images of the vasculature.

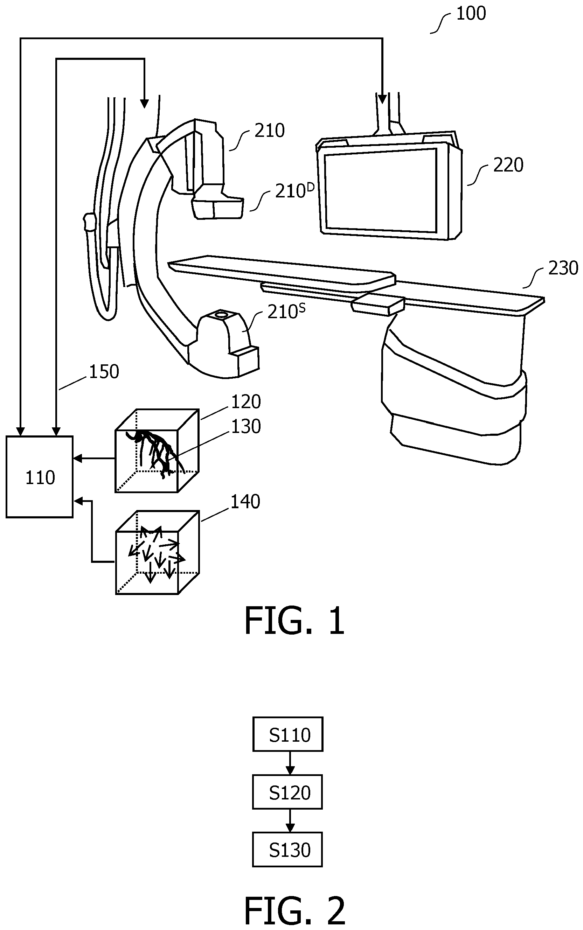

Fig. 1 is a schematic diagram illustrating an example of a system 100 for providing a viewing angle for acquiring projection data of a vascular region, in accordance with some aspects of the present disclosure. Fig. 2 is a flowchart illustrating an example of a computer-implemented method of providing a viewing angle for acquiring projection data of a vascular region, in accordance with some aspects of the present disclosure. It is noted that operations that are described as being performed by the one or more processors 110 of the system 100 illustrated in Fig. 1, may also be performed in the method illustrated in Fig. 2. Likewise, operations that are described in relation to the method described with reference to Fig. 2 may also be performed by the one or more processors 110 of the system 100 illustrated in Fig. 1. With reference to Fig. 1, and Fig. 2, the system 100 for providing a viewing angle for acquiring projection data of a vascular region includes one or more processors 110 configured to:
receive S110 volumetric data 120 representing the vascular region 130;
determine S120, using the volumetric data 120, and based on a simulated motion 140 of the vascular region 130 and/or based on a motion of the vascular region 130 represented within the volumetric data 120, a value of a viewing angle α₁, β₁ for acquiring projection data 150 representing the vascular region 130; and
output S130 the value of the viewing angle α₁, β₁.

The inventors have observed that a drawback of known techniques for determining viewing angles for acquiring projection data of the vasculature is that they are optimized under the assumption that the shape, and also the position, of the vasculature is static. Such viewing angles may be optimal for acquiring projection data of the vasculature when the actual shape and position of the vasculature corresponds to the assumed, static, shape and position. However, in practice, the vasculature is typically subjected to various types of motion, and this motion changes the shape, and also the position, of the vasculature. The source of the motion may be cardiac motion, respiratory motion, bulk motion of the anatomy, and so forth. The failure to account for such motion gives rise to projection X-ray images that are sub-optimal. The resulting projection X-ray images may even be misleading due to vessel overlaps, and also increased amounts of vessel foreshortening. Since the system described above determines a viewing angle for acquiring projection data using volumetric data representing the vascular region based on a simulated motion of the vascular region, and/or based on a motion of the vascular region represented within the volumetric data, the system takes account of changes in the shape and position of the vasculature and consequently provides a more optimal value of the viewing angle. The use of the viewing angle provided by the system therefore reduces the risk of acquiring projection X-ray images that give a misleading impression of the vasculature.

The operations that are performed by the processor(s) 110 of the system 100 are described in more detail below.

In the operation S110, volumetric data 120 is received. The volumetric data 120 represents a vascular region 130.

The volumetric data 120 may represent various vascular regions. The volumetric data 120 may represent a portion of the cardiac vasculature, for example. Alternatively, the volumetric data 120 may represent a portion of the vasculature from another anatomical region than the heart. For example, the volumetric data 120 may represent a portion of the pulmonary vasculature, the peripheral vasculature, the vasculature of the brain, and so forth. In general, the vascular region may include one or more vessels. The vascular region may include one or more vessels such as arteries, veins, capillaries, and so forth. The volumetric data that is received in the operation S 110 may be generated by various types of imaging systems. These include various volumetric imaging system such as computed tomography "CT" imaging systems, magnetic resonance imaging "MRI" systems, and 3D ultrasound imaging systems. The CT imaging system may be a spectral CT imaging system. In contrast to conventional CT imaging systems, and which generate X-ray attenuation data representing X-ray attenuation within a single energy interval, spectral CT imaging systems generate X-ray attenuation data representing X-ray attenuation within multiple different energy intervals. The X-ray attenuation data generated by a spectral CT imaging system may be processed, e.g. using various material decomposition algorithms, in order to distinguish between media that have similar X-ray attenuation values when measured within a single energy interval, and which would be indistinguishable in X-ray attenuation data obtained using a conventional CT imaging system. Thus, the X-ray attenuation data generated by spectral CT imaging systems may be processed to provide volumetric images with improved specificity to materials such as contrast agent, tissue, bone, and so forth.

In one example, the volumetric data that is received in the operation S 110 is generated by a (spectral) projection X-ray imaging system. The (spectral) projection X-ray imaging system may be configured to acquire projection data representing the vascular region from multiple viewing angles with respect to the vascular region 130. A tomographic reconstruction of the projection data from the multiple viewing angles is then performed in order to provide volumetric data representing the vascular region 130. A volumetric image that is obtained using a (spectral) projection X-ray imaging system in this manner is sometimes referred-to as a 3D rotational angiogram "3DRA".

As described in more detail in the examples below, in some examples, the volumetric data 120 that is received in the operation S 110 comprises a single volumetric image representing the vascular region 130. The single volumetric image may represent the vascular region at a point in time. In general, the point in time may be any point in time. In some examples, the point in time is a specific point in time. For instance, the point in time may correspond to a specific point in the cardiac cycle, or a specific point in the respiratory cycle. By way of some examples, the specific point in the cardiac cycle may be the End Diastolic "ED" cardiac phase, of the End Systolic "ES" phase, and the specific point in the respiratory cycle may be the fully-inhaled respiratory state, or the fully-exhaled respiratory state. In other examples, the volumetric data 120 that is received in the operation S 110 comprises a plurality of volumetric images representing the vascular region 130. For example, the volumetric data 120 may comprise a temporal sequence of volumetric images representing the vascular region 130. The temporal sequence of volumetric images may represent the vascular region at a plurality of corresponding points in the cardiac cycle, or at a plurality of corresponding points in the respiratory cycle, for example.

In some examples, the volumetric data 120 that is received in the operation S110 is generated subsequent to the injection of a contrast agent into the vasculature of a patient. In these examples, the volumetric data 120 represents a distribution of the contrast agent in the vascular region 130. A contrast agent serves to improve the distinction between blood and surrounding tissue in the volumetric data 120. In these examples, the volumetric data 120 may be referred-to as angiographic volumetric image data 120. Angiographic volumetric image data may be generated by a (spectral) CT imaging system, or by an MRI system. An example of angiographic volumetric image data that is generated by a CT imaging system is a Cardiac Computed Tomography Angiography "CCTA" image.

The volumetric data 120 may be received from various sources in the operation S110. For example, the volumetric data 120 may be received from a medical imaging system such as one of the imaging systems described above. Alternatively, the volumetric data 120 may be received from another source, such as a computer readable storage medium, the Internet, the Cloud, and so forth. In general, the volumetric data 120 may be received by the one or more processors 110 via any form of data communication. The volumetric data 120 may be received via wired, or wireless, or optical fiber communication, for example. By way of some examples, when wired data communication is used, the communication may take place via electrical signals that are transmitted on an electrical cable. When wireless data communication is used, the communication may take place via RF or infrared signals. When an optical fiber data communication is used, the communication takes place via optical signals that are transmitted on an optical fiber.

Referring now to the operation S120 mentioned above with reference to Fig. 1 and Fig. 2; in this operation, a value of a viewing angle α₁, β₁ for acquiring projection data 150 representing the vascular region 130, is determined.

In a first set of examples, the value of the viewing angle α₁, β₁ is determined using the volumetric data 120, and based on a simulated motion 140 of the vascular region 130. In a second set of examples, the value of the viewing angle α₁, β₁ is determined using the volumetric data 120, and based on a motion of the vascular region 130 represented within the volumetric data 120. Combinations of these examples are also contemplated.

Taking, initially, the first set of examples and in which the value of the viewing angle α₁, β₁ is determined using the volumetric data 120, and based on a simulated motion 140 of the vascular region 130. In these examples, the operation of determining the value of the viewing angle α₁, β₁ may include:
analyzing the volumetric data 120 to determine a volumetric shape of the vascular region 130 at a point in time
determining, based on a simulated motion 140 of the vascular region 130, a simulated volumetric shape of the vascular region 130 at each of one or more different points in time; and
for each of a plurality of candidate viewing angles α_{i⊂1..m}, β_{j⊂1..m}:
evaluating a vessel overlap metric and/or a vessel foreshortening metric for at least a portion of the simulated volumetric shape of the vascular region 130 for the one or more different points in time; and
determining the value of the viewing angle α₁, β₁ based on the vessel overlap metrics and/or the vessel foreshortening metrics corresponding to the one or more different points in time for the candidate viewing angles α_{i⊂1..m}, β_{j⊂1..m}.

In the first set of examples, the operation of analyzing the volumetric data 120 to determine a volumetric shape of the vascular region 130 at a point in time, may include generating a vascular model representing the vascular region. Various known image segmentation techniques, neural networks, and path-finding algorithms may be used to generate the vascular model. In general, the vascular model represents the path(s) of the vessel(s) in the vascular region. The vascular model may represent the centerline(s) of the path(s) of the vessel(s) for example. The vascular model may alternatively represent the vessel(s) themselves, e.g. in the form of segmented vessel(s), such that the path(s) of the vessel(s) are inherently represented by the vessel(s). In general, the point in time may be any point in time, as described above. In one example, the volumetric data comprises a single static image representing a vascular region at a point in time, and the volumetric shape of the vascular region 130 that results from the analyzing operation represents the shape of the vascular region at the same point in time.

In the first set of examples, a simulated motion 140 of the vascular region 130, is then used to determine a simulated volumetric shape of the vascular region 130 at each of one or more different points in time. The one or more different points in time are different to the point in time for which the volumetric shape of the vascular region 130 was determined in the analyzing operation. The one or more different points in time may correspond to different points in the cardiac cycle, or to different points in the respiratory cycle. In one example, the simulated motion 140 of the vascular region is used to provide a simulated volumetric shape of the vascular region 130 at multiple points in time that span a complete cardiac cycle, or at multiple points in time that span a complete respiratory cycle.

In one example, the simulated motion 140 of the vascular region is determined using a parametric motion model. In this example, the one or more processors 110 are configured to:
simulate the motion of the vascular region 130 using a parametric motion model, to provide the simulated motion 140 of the vascular region 130.

A parametric motion model is a representation of the motion of the vascular region 130. In one example, the vascular region is a cardiovascular region, and the parametric motion model represents the motion of the heart over at least a portion of a cardiac cycle.

The parametric motion model may take various forms. For example, the parametric motion model may represent the motion of the vascular region via the displacement of points in cartesian space (*x*, *y*, z) in the vascular region over time. For example, in one implementation, a displacement vector field (*mₓ, m_{y}, m_{z}*) wherein each entry is a 3D vector describes the motion of points in the vascular region over time. The displacement vector fields for a data-driven parametric motion model of the heart may be obtained from an imaging modality such as 3D ultrasound. The parametric motion model may represent different aspects of cardiac motion. For instance, it may represent cardiac contraction, cardiac torsion, respiratory motion, and so forth. The displacement vector field may be used to determine the motion of the points in cartesian space over time, i.e. as a function of (*x, y, z, t*)*.* The parametric motion model may represent the motion in an analytic, or a data-driven form. For instance, in the example of a cardiovascular region, a data-driven form of a parametric model may include displacement vector fields that are defined for a discrete set of cardiac phases [*c*₀,..,*c_{N}*], and which indicate the displacements of points on the cardiac surface from a cardiac phase *cᵢ* to a cardiac phase *c*_{*i*+1}. An example for a hybrid model is a set of displacement vector fields indicating the displacements of points on the heart surface after cardiac contraction from the ED cardiac phase to the ES cardiac phase, and wherein the length of the vectors in the displacement vector field is modulated for each cardiac phase *cᵢ* by an analytic function.

The operation of simulating the motion of the vascular region 130 using the parametric motion model, may be performed by using the displacement vector fields of the parametric motion model to deform the vascular model described above that resulted from analyzing the volumetric data 120. For example, in order to obtain a motion trajectory for a vascular tree, the vessels, or the vessel centerlines described above, may be deformed by their corresponding displacement vector fields. This operation may include extrapolating the displacement vector fields represented by the parametric motion model such that the size and shape of the parametric motion model corresponds to the size and shape of the vasculature in the vascular region 130. For instance, the parametric motion model may represent an average model for multiple different patients, and the displacement vector fields represented by the average parametric motion model may be extrapolated in order to fit the average parametric motion model to the vasculature of a given patient. In the cardiovascular example described above, the parametric motion model may be applied n-times to the cardiovascular region, resulting in N deformed cardiovascular trees, and which correspond to N cardiac phases. The parameter N is set according to the desired granularity of the motion.

In one example, the parametric motion model includes one or more patient parameters and the one or more processors 110 are configured to simulate the motion 140 of the vascular region 130 using the patient parameters.

The patient parameters may control aspects of the simulated motion such as the extent of the motion (e.g. the extent of contraction of the heart, as determined from the ejection fraction for the subject), the size of the modelled vascular region, and the motion cycle. Other examples of patient parameters for the parametric motion model include indicators for the presence of disease patterns like arrythmia. Such parameters may be used to select a parametric motion model that provides a motion pattern that is closest to the expected motion in a given patient.

In one example, the one or more processors 110 also analyze the volumetric data 120 to determine the motion of the vascular region 130. In this example, the volumetric data 120 that is received in the operation S 110 comprises a temporal sequence of volumetric images representing the vascular region 130. The temporal sequence of images inherently represent the motion of the vascular region via changes in the shape and/or position of vessel(s) in the vascular region over time. The motion, as derived from the volumetric data, may be used to fine-tune the motion represented by the parametric motion, to that of the vascular region. For example, it may be used to simulate a condition such as cardiac arrythmia.

In another example, the one or more processors 110 are configured to:
analyze the volumetric data 120 to determine a motion state of the vascular region 130.

This example may similarly be implemented in situations wherein the volumetric data 120 that is received in the operation S 110 comprises a temporal sequence of volumetric images representing the vascular region 130. In this example, the motion state may refer to a cardiac phase (e.g. ED cardiac phase, ES cardiac phase, and so forth), a respiratory phase (e.g. fully inhaled, fully exhaled), or another state of the motion. The motion state may be determined from the volumetric data by using image analysis techniques to monitor the movement of anatomical features, such as the opening and closing of a cardiac valve, the motion of the aorta, the motion of the diaphragm, and so forth. In this example, known image processing techniques such as those described in a document by Lessick, J., et al., "Automatic determination of differential coronary artery motion minima for cardiac computed tomography optimal phase selection", Acad. Radiol. 2015 Jun;22(6):697-703, may be used to identify different phases of the cardiac cycle. The motion state, in this case the identified cardiac phase(s) of the volumetric data, may then be used to label the volumetric data.

Instead of analyzing the volumetric data 120 to determine a motion state of the vascular region 130, the motion state may be derived from sensor data that is acquired contemporaneously with the volumetric data 120. In this case, the one or more processors may receive sensor data representing a motion state of the vascular region 130. Various types of sensors may provide the sensor data. For example, an ECG sensor may provide sensor data representing a cardiac phase, or a chest band may provide sensor data representing a respiratory phase.

The motion state of the vascular region 130, as determined from the volumetric data 120, or from the received sensor data, may then be used by the one or more processors 110 to determine the value of the viewing angle α₁, β₁. For instance, if the volumetric data 120 that is received in the operation S110 comprises a single volumetric image representing the vascular region 130, the motion state may be used to synchronize the motion state of the vascular region 130 to the parametric motion model. Similarly, if the volumetric data 120 that is received in the operation S 110 comprises a temporal sequence of volumetric images representing the vascular region 130, the motion state that is determined by analyzing the volumetric data 120 may be used to synchronize the parametric motion model to the motion state of the vascular region.

In some situations, a user may wish to determine an optimal viewing angle for continuously-acquired projection data. For example, in an interventional navigation situation, a user may obtain projection data of a cardiovascular region over the complete cardiac cycle. In such situations, the user may desire a viewing angle that is optimal for viewing a vascular region over the complete cardiac cycle. Here, the motion state that is determined from the volumetric data, or from the sensor data, may be used to synchronize the parametric motion model to the motion state of the vascular region, and consequently to simulate the motion of the vascular region at multiple cardiac states that span the complete cardiac cycle. An optimum viewing angle may then be determined that is on average, optimal for acquiring projection data over the complete cardiac cycle.

In other situations, a user may wish to determine an optimal viewing angle for a specific point in the cardiac/ respiratory cycle, or for a specific portion of the cardiac/ respiratory cycle. For example, a user may wish to determine the optimal viewing angle viewing a cardiovascular region in the ED cardiac phase, or for the duration of the diastole cardiac phase. The motion state that is determined from the volumetric data, or from the sensor data, may be used to support these situations by using the motion state to synchronize the parametric motion model to the motion state of the vascular region 130, and consequently to determine the shape of the vascular region in different motion state(s), or over a range of different motion states. An optimum viewing angle may then be determined that is optimal for acquiring projection data in the different motion state, or which is on average, optimal over the range of motion states.

In a related example, the one or more processors 110 are configured to:
receive input data specifying a motion state; and
wherein the one or more processors 110 are configured to determine the value of the viewing angle α₁, β₁ for the specified motion state.

This example may be used in the situations described above and in which a user wishes to determine an optimal viewing angle for a specific point in the cardiac/ respiratory cycle. In this example, the motion state, as determined from the received input data is used to determine, based on a simulated motion 140 of the vascular region 130, a simulated volumetric shape of the vascular region 130 at that point in time. The viewing angle is then optimized for that simulated volumetric shape of the vascular region 130.

In one example, the one or more processors 110 are configured to:
analyze the volumetric data 120 to provide a vascular model representing a volumetric shape of the vascular region 130 and/or an anatomical model representing an anatomical region coupled to the vascular region 130; and
wherein the simulated motion 140 of the vascular region 130, is determined using the vascular model and/or the anatomical model, respectively.

This example may be used in the operation of determining the simulated volumetric shape of the vascular region 130 at each of one or more different points in time, that was described above. The vascular model provides the shape of the vasculature that is subsequently deformed using the parametric motion model. The vascular model may represent the volumetric shape of the vascular region 130 via a 3D representation of the centerline(s) of one or more vessels in the vascular region, for example. The anatomical model may provide useful information for fine-tuning the motion of the vascular region that is simulated using the parametric motion model. In this example, the operation of analyzing of the volumetric data 120 to provide the vascular model may be performed using the various known image segmentation techniques, neural networks, and path-finding algorithms, as described above. The anatomical model may be obtained from the volumetric data using the analysis techniques described above, i.e. via segmentation, or via the use of a trained neural network. The anatomical model may represent a portion of the anatomy such as a portion of cardiac surface, or a portion of a lung, and which is coupled to the vascular region, for example. For instance, an anatomical model representing a cardiac surface, may be used to provide motion data that is used to fine-tune the motion of the vascular region that is simulated using the parametric motion model.

After having determined a simulated volumetric shape of the vascular region 130 at each of one or more different points in time, the simulated volumetric shape(s) of the vascular region 130 are used to evaluate a vessel overlap metric and/or a vessel foreshortening metric for at least a portion of the simulated volumetric shape of the vascular region 130 for the one or more different points in time; for each of a plurality of candidate viewing angles α_{i⊂1..m}, β_{j⊂1..m}. The vessel overlap metric and the vessel foreshortening metric may be evaluated in the manner described below.

In one example, the operation of evaluating a vessel overlap metric and/or a vessel foreshortening metric for at least a portion of the simulated volumetric shape of the vascular region 130 for the one or more different points in time, comprises:
projecting the simulated volumetric shape of the vascular region 130 for the one or more different points in time, from the candidate viewing angle α_{i⊂1..m}, β_{j⊂1..m}, to provide a virtual projection of the simulated volumetric shape of the vascular region 130 for the one or more different points in time; and
evaluating the vessel overlap metric and/or the vessel foreshortening metric for the at least a portion of the vascular region 130 for the one or more points in time based on the virtual projections of the simulated volumetric shapes of the vascular region 130.

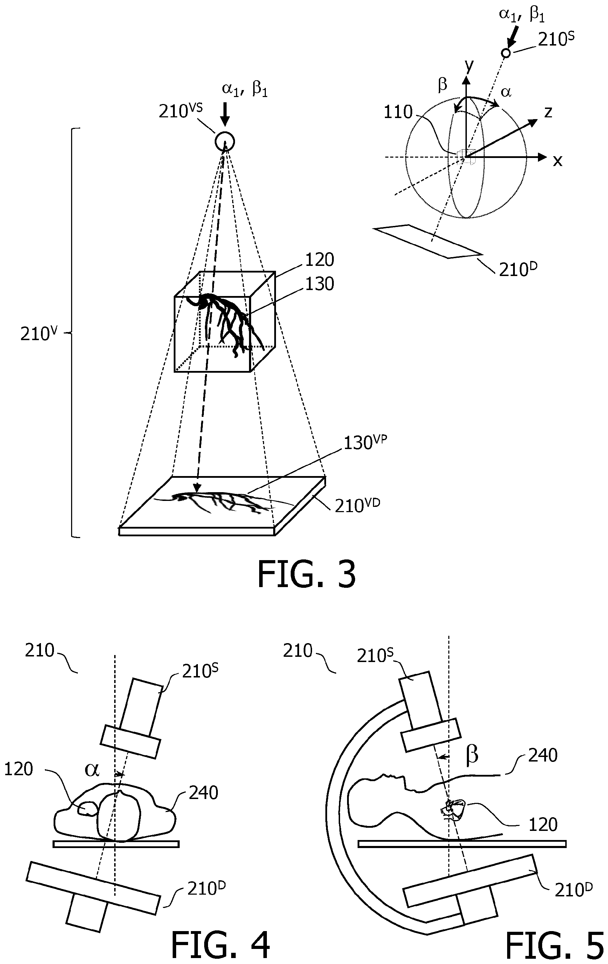

The generation of these virtual projections is now described with reference to Fig. 3 - Fig. 5. Fig. 3 is a schematic diagram illustrating an example of the generation of a virtual projection 130^{VP} of a vascular region 130 from a viewing angle α₁, β₁, of a virtual projection X-ray imaging system 210^{V} with respect to a vascular region 130, in accordance with some aspects of the present disclosure. The virtual projection X-ray imaging system 210^{V} illustrated in Fig. 3 includes virtual X-ray source 210^{VS} and a virtual X-ray detector 210^{VD}. The geometry of the virtual X-ray source 210^{VS} and the virtual X-ray detector 210^{VD} corresponds to the geometry of the X-ray source 210^{S} and the X-ray detector 210^{D} of the projection X-ray imaging system 210 illustrated in Fig. 1 that will subsequently be used to acquire the projection data. For instance, the shapes of the beams of X-ray radiation emitted by the X-ray sources 210^{S} and 210^{VS} correspond to one another, the areas of the X-ray detectors 210^{D} and 210^{VD} correspond to one another, and the relative positioning of the X-ray sources 210^{S} and 210^{VS} and X-ray detectors 210^{D} and 210^{VD} in relation to the vascular region 130 correspond to one another. In this example, the vascular region 130 is represented by the volumetric data 120. As described above, the volumetric data 120 may be generated by a volumetric imaging system, or alternatively it may be provided by a 3DRA. The virtual projection X-ray imaging system 210^{V} illustrated in Fig. 3 has a viewing angle with respect to the vascular region 130. The viewing angle may be defined by the orientation of a central ray of the virtual X-ray source 210^{VS} with respect to the vascular region 130. In the example illustrated in Fig. 3, the viewing angle is defined by the angle α and the angle β with respect to the vascular region, although in general, the viewing angle may be defined by one or more such angles.

The angles α, and β, may be defined with respect to the vascular region 130 by reference to various anatomical axes of a patient 240. For instance, the angle α is defined with respect to the ventral-dorsal axis, as illustrated in Fig. 4, which is a schematic diagram illustrating a first example of a viewing angle α of a projection X-ray imaging system 210 with respect to a vascular region 130, in accordance with some aspects of the present disclosure. The angle β is defined with respect to the cranial-caudal axis, as illustrated in Fig. 5, which is a schematic diagram illustrating a second example of a viewing angle β of a projection X-ray imaging system 210 with respect to a vascular region 130, in accordance with some aspects of the present disclosure. The viewing angle may alternatively be defined with respect to other anatomical axes. The viewing angle may also include a definition of a rotational angle of a central ray of the virtual X-ray source 210^{VS} with respect to the anatomy.

The virtual X-ray source 210^{VS} and the virtual X-ray detector 210^{VD} illustrated in Fig. 3 may be used to generate a virtual projection 130^{VP} of a vascular region 130 from the viewing angle by projecting, in a mathematical sense, the volumetric data 120 representing the vascular region 120 onto the virtual X-ray detector 210^{VD} using virtual X-rays emitted from the virtual X-ray source 210^{VS}. For instance, at the viewing angles defined by α₁, and β₁, portions of the vascular region 130 lying along the path of the virtual X-ray indicated by the dashed arrowed line in Fig. 3 are projected onto the virtual detector 210^{VD} by calculating a line integral of the intensity values represented by the volumetric data 120 that lie along the path of the virtual X-ray to provide an image intensity value of a point in the virtual projection 130^{VP} at which the virtual X-ray intersects virtual X-ray detector 210^{VD}. The same operation may be repeated for multiple virtual X-rays that intersect different points across the virtual X-ray detector 210^{VD} to generate the virtual projection 130^{VP} of the vascular region 130.

In one example, the simulated volumetric shape of the vascular region 130 for the one or more different points in time, is projected from multiple candidate viewing angles α_{i⊂1..m}, β_{j⊂1..m}, to provide a virtual projection of the simulated volumetric shape of the vascular region 130 for the one or more different points in time. The value of the viewing angle α₁, β₁ is then determined based on the virtual projections of the simulated volumetric shapes of the vascular region 130 corresponding to the one or more different points in time for the candidate viewing angles α_{i⊂1..m}, β_{j⊂1..m}.

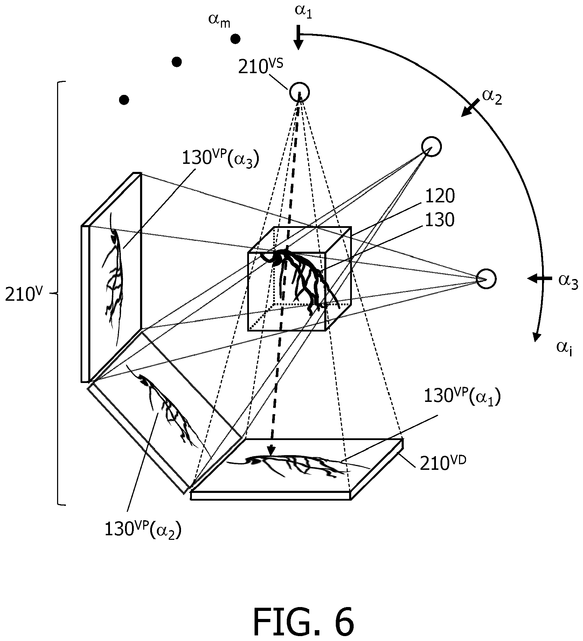

By way of an example, Fig. 6 is a schematic diagram illustrating an example of the generation of virtual projections 130^{VP}(αᵢ) of a vascular region 130 from corresponding viewing angles αᵢ of a virtual projection X-ray imaging system 210^{V} with respect to a vascular region 130, in accordance with some aspects of the present disclosure. For each viewing angle αᵢ, the corresponding virtual projection 130^{VP}(αᵢ), is illustrated. The virtual projections 130^{VP}(αᵢ) are generated using the projection technique described above. It is noted that in the example illustrated in Fig. 6, the virtual projections 130^{VP}(αᵢ) are shown for a single, different, point in time. If projections are generated for multiple points in time, then multiple virtual projections are generated at each viewing angle αᵢ. It is also noted that whilst the viewing angles in the example illustrated in Fig. 3 have discrete values αᵢ, the viewing angles may alternatively assume continuously-variable values. In his example, the value of the viewing angle α₁, β₁ is determined based on the virtual projections of the simulated volumetric shapes of the vascular region 130 corresponding to the one or more different points in time for the candidate viewing angles α_{i⊂1..m}, β_{j⊂1..m}.

In one example, the one or more processors 110 are configured to determine the value of the viewing angle α₁, β₁ based on a minimum vessel overlap criterion for at least a portion of the vascular region 130. The minimum vessel overlap criterion and/or the minimum vessel foreshortening criterion is evaluated based on a calculated value of a respective vessel overlap metric and/or a vessel foreshortening metric for the at least a portion of the vascular region 130 at each of a plurality of candidate viewing angles α_{i⊂1..m}, β_{j⊂1..m}.

A vessel overlap metric may be calculated in various ways. For instance, a vessel overlap metric may represent the total number of overlapping vessels, or the total area of vessel overlap regions, for the vessels in the at least a portion of the vascular region 130. The overlap metric may be evaluated by casting virtual rays in the projection direction through the vascular region 130, and counting the number of intersections with vessels. The minimum vessel overlap criterion may be satisfied at a viewing angle α₁, β₁ at which these exists a minimum amount of vessel overlap; e.g. there exists a minimum total number of overlapping vessels, or a minimum total area of vessel overlap regions, for the vessels in the at least a portion of the vascular region 130.

In a related example, the one or more processors 110 are configured to evaluate the minimum vessel overlap criterion based on a virtual projection 130^{VP} of the vascular region 130 from each of the plurality of candidate viewing angles α_{i⊂1..m}, β_{j⊂1..m}.

The generation of the virtual projections in accordance with this example was described above with reference to Fig. 3 - Fig. 6.

In another example, instead of, or in addition to, determining the value of the viewing angle α₁, β₁ based on a minimum vessel overlap criterion, the one or more processors 110 are configured to determine the value of the viewing angle α₁, β₁ based on a minimum vessel foreshortening criterion for at least a portion of the vascular region 130. The minimum vessel overlap criterion and/or the minimum vessel foreshortening criterion is evaluated based on a calculated value of a respective vessel overlap metric and/or a vessel foreshortening metric for the at least a portion of the vascular region 130 at each of a plurality of candidate viewing angles α_{i⊂1..m}, β_{j⊂1..m}.

A vessel foreshortening metric may be calculated in various ways. For instance, a vessel foreshortening metric may represent a (combined) length of one or more vessel segments in the at least a portion of the vascular region 130 when viewed from the viewing angle α₁, β₁. The (combined) length of the one or more vessel segments can be calculated explicitly, e.g. based on the length(s) of projections of the vessel segment(s), or alternatively a surrogate measure of the combined length may be calculated. For example, a surrogate measure of the combined length may be calculated by evaluating the inner products of the tangential centerline vectors of one or more vessel segments in the vascular region with their corresponding projection directions. The minimum vessel foreshortening criterion may be satisfied at a viewing angle α₁, β₁ at which there is minimum vessel foreshortening; e.g. there exists a maximum (combined) length of the one or more vessel segments in the at least a portion of the vascular region 130.

In one example, the one or more processors 110 are configured to evaluate the minimum vessel foreshortening criterion based on a virtual projection 130^{VP} of the vascular region 130 from each of the plurality of candidate viewing angles α_{i⊂1..m}, β_{j⊂1..m}. In this example, a virtual projection is generated for each candidate viewing angle, and a value of the vessel foreshortening metric is evaluated from the virtual projections. The minimum vessel foreshortening criterion is evaluated by comparing the vessel foreshortening metrics for the different candidate viewing angles. The generation of the virtual projections in accordance with this example was described above with reference to Fig. 3 - Fig. 6.

In another example, instead of evaluating the minimum vessel foreshortening criterion based on virtual projections of the vascular region, the minimum vessel foreshortening criterion is instead evaluated based on the inner products of the tangential centerline vectors of one or more vessel segments in the vascular region with their corresponding projection directions. The projection direction is the direction in which a vessel segment is projected for a given candidate viewing angle, and is determined by the position of the vessel segment within a beam (e.g. a cone) of virtual X-rays emitted by the X-ray source. By way of an example, the one or more vessels in the vascular region 130 may be divided into vessel segments that are each represented by a tangential centerline vector. For each of a plurality of candidate viewing angles α_{i⊂1..m}, β_{j⊂1..m}, a projection direction corresponding to each vessel segment is determined based on the position of the segment within the beam of virtual X-rays emitted by the X-ray source, and the inner product is calculated between the tangential centerline vector for the segment, and the corresponding projection direction. Both vectors may be normalized to unit length before computing the inner product. The higher the value of the inner product for the segment, the more parallel the vessel segment is to the projection direction, and consequently the higher the amount of foreshortening. The values of the inner products for the segments are then combined (e.g. summed) to provide a foreshortening metric for the vessel segment(s) in the vascular region for the candidate viewing angle. In this example, the minimum vessel foreshortening criterion is met at the candidate viewing angle α_{i⊂1..m}, β_{j⊂1..m} for which the foreshortening metric has the lowest value.

If a combination of the minimum vessel overlap criterion and the minimum vessel foreshortening criterion is used to determine the value of the viewing angle α₁, β₁, weights may be applied to each of the vessel overlap metric and the vessel foreshortening metric, and the weights used to evaluate a combined metric for use in determining the value of the viewing angle α₁, β₁. In general, the at least a portion of the vascular region 130 may be a portion, or all, of one or more vessels in the vascular region. For example, it may be a portion of a cardiac artery, or an entire cardiac artery, such as the Left Anterior Descending artery.

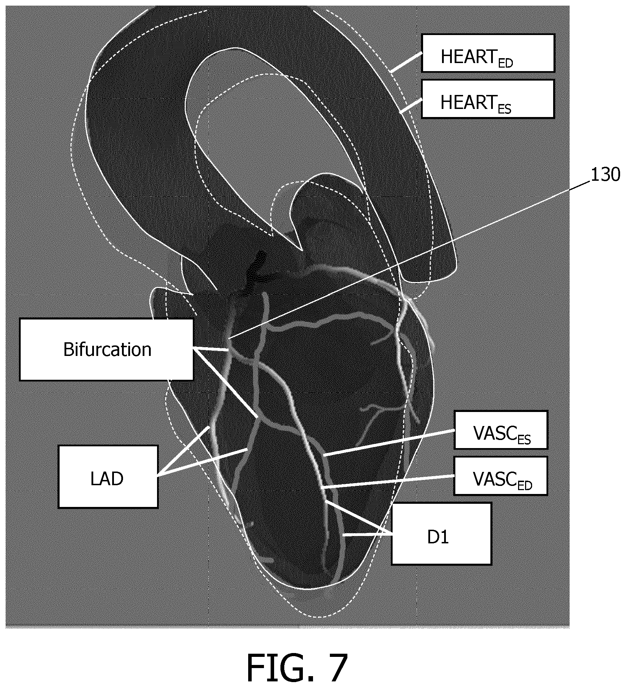

An example of the operation of determining the value of the viewing angle α₁, β₁ based on a minimum vessel foreshortening criterion is now described with reference to Fig. 7 - Fig. 9. Fig. 7 is a schematic diagram illustrating the effect of cardiac motion on vessel foreshortening, in accordance with some aspects of the present disclosure. Fig. 7 illustrates the outline of a heart, in each of two cardiac phases. The outline HEART_{ED} illustrates the outline of the heart in the ED cardiac phase, i.e. when the heart atria and ventricles are relaxed and have been filled with blood. The outline HEART_{ES} illustrates the outline of the heart in the ES cardiac phase, i.e. after the atria have contracted and the blood has been pumped from the heart. The shapes of the cardiac vasculature corresponding to each of these cardiac phases are also illustrated in Fig. 7. The label VASC_{ED} illustrates various cardiac vessels in the ED cardiac phase, and the label VASC_{ES} illustrates the same cardiac vessels in the ES cardiac phase. The cardiac vessels illustrated in Fig. 7 include the Left Anterior Descending "LAD" artery labelled "LAD", the first diagonal branch of the LAD artery labelled "D1", and the bifurcation between the LAD and D1 labelled "Bifurcation". For reference, the cardiac vessels in the ED cardiac phase are shaded to indicate the distance from the ostium to points along these vessels.

As can be seen in Fig. 7, the heart, and consequently the cardiac vasculature, undergoes significant motion in the period between the ED cardiac phase, i.e. the end of the relaxed state, and the ES phase, i.e. the end of the contracted state. This motion affects both the shape, and also the position of the cardiac vessels. This, in-turn, affects the optimum viewing angle for viewing portions of the cardiac vessels because it affects vessel overlaps, and also vessel foreshortening, as described above. The motion may affect the optimum viewing angle for viewing the bifurcation labelled "Bifurcation", the Left Anterior Descending "LAD" artery labelled "LAD", and the first diagonal branch of the LAD artery labelled "D1", in Fig. 7, for example.

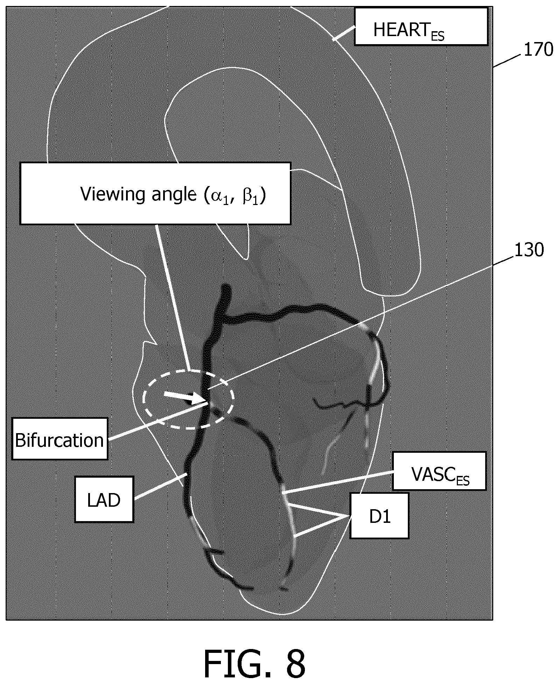

An example of a viewing angle that has been optimized based on a minimum vessel foreshortening criterion, is now described with reference to Fig. 8. Fig. 8 is a schematic diagram illustrating an example of a graphical representation 170 of a vascular region 130 including an example of a viewing angle α₁, β₁, that has been optimized for minimum foreshortening around the LAD-D1 bifurcation in the ES cardiac phase, in accordance with some aspects of the present disclosure. In the example illustrated in Fig. 8, the amount of foreshortening in the labelled cardiac vessels has been found to have a minimum value at the viewing angle α₁, β₁. The minimum vessel foreshortening criterion has been evaluated based on a virtual projection of the vascular region 130 from each of a plurality of candidate viewing angles. For the optimum viewing angle α₁, β₁, the amount of foreshortening is illustrated via the shading of the vasculature in the ES cardiac phase labelled VASC_{ES}. In the illustrated example, the amount of foreshortening is calculated as the difference between the distance from the ostium to a point in the vasculature in the volumetric data, i.e. a point in a volumetric image of the vascular region, and the distance from the ostium to the same point in the vasculature in a virtual projection of the vascular region that is generated by projecting the vascular region from the viewing angle α₁, β₁, using a virtual X-ray projection imaging system. The generation of the virtual projection was described above with reference to Fig. 3 - Fig. 5.

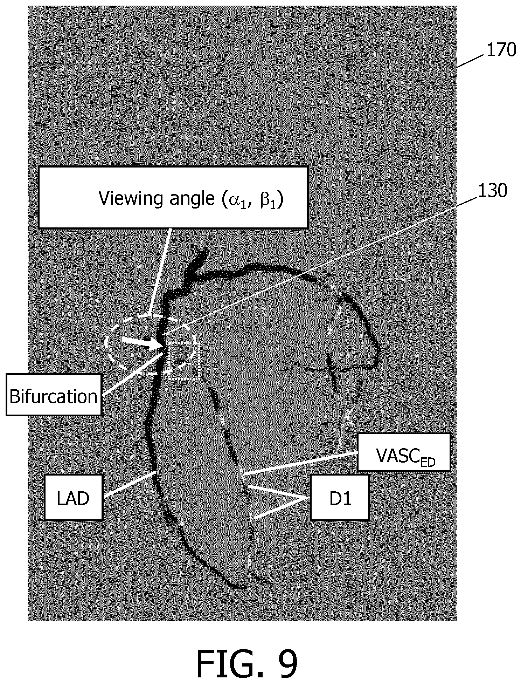

In order to highlight the benefit of evaluating the optimum viewing angle α₁, β₁, for a specific point in the cardiac cycle, an example is now described wherein the amount of foreshortening is evaluated for the same viewing angle α₁, β₁, as that illustrated in Fig. 8, but at a different point in the cardiac cycle. Fig. 9 is a schematic diagram illustrating an example of a graphical representation 170 of a vascular region 130 including an indication of the amount of foreshortening observed in the ED cardiac phase for a viewing angle α₁, β₁, that has been optimized for minimum foreshortening around the LAD-D1 bifurcation in the ES cardiac phase, in accordance with some aspects of the present disclosure. Items in Fig. 8 that share the same labels as items in Fig. 9 refer to the same item. In contrast to Fig. 8, Fig. 9 illustrates the amount of foreshortening for the cardiac vessels in the ED cardiac phase, as indicated by the label VASC_{ED}. As may be observed in Fig. 9, within the rectangular-shaped dotted box, the amount of foreshortening in the proximal portion of the first diagonal branch of the LAD artery labelled "D1" in Fig. 9 has increased substantially in comparison to the amount of foreshortening in the same position in Fig. 8. Consequently, the optimum viewing angle α₁, β₁, that has been evaluated for the ES cardiac phase, is no longer optimal for the ED cardiac phase. This example highlights the benefit of evaluating the optimum viewing angle α₁, β₁, for a specific point in the cardiac cycle.

As may be appreciated, the minimum vessel overlap criterion may be evaluated based on a virtual projection of the vascular region 130 from each of a plurality of candidate viewing angles in a similar manner to that described with reference to Fig. 7. In this case, the vessel overlap metric is evaluated at each of the plurality of candidate viewing angles.

Thus, the value of the viewing angle α₁, β₁ is determined based on the value of the vessel overlap metric and/or the vessel foreshortening metric, for the at least a portion of the vascular region 130 for each candidate viewing angle α_{i⊂1..m}, β_{j⊂1..m}, using the corresponding virtual projection 130^{VP}. If the value of the viewing angle α₁, β₁ is to be determined for a point in time, e.g. a point in the cardiac/ respiratory cycle, this operation involves determining a viewing angle at which the vessel overlap metric and/or the vessel foreshortening metric has a minimum value. If both criteria are applied, weights may be applied to each of the vessel overlap metric and the vessel foreshortening metric, and the weights used to evaluate a combined metric that is then used to determine the value of the viewing angle α₁, β₁ for which the combined metric has a minimum value. By way of an example, the total number of overlapping vessels, or the (combined) length of one or more vessel segments, may be calculated for the at least a portion of the vascular region 130 using the virtual projection 130^{VP}. The minimum vessel overlap criterion and/or the minimum vessel foreshortening criterion are then applied by determining from the candidate viewing angles, a viewing angle for which the total number of overlapping vessels is minimum, or the (combined) length of one or more vessel segments is minimum

If the value of the viewing angle α₁, β₁ is to be determined for a portion of a cardiac/ respiratory cycle, or for the complete cardiac/ respiratory cycle this operation involves determining a viewing angle at which the vessel overlap metric and/or the vessel foreshortening metric has a minimum value over the relevant part of the cardiac/ respiratory cycle. In this case, the individual metrics may be combined for the different points in the cardiac/ respiratory cycle to provide an overall metric for the relevant part of the cardiac/ respiratory cycle. The overall metric may represent the weighted average of the individual metrics for the different points in the cardiac/ respiratory cycle. The weighting may balance the scale across the individual metrics, or emphasize any of them, as desired by the user or clinical application.

The candidate viewing angles for which the vessel overlap metric is evaluated may be determined in various ways. For instance, the candidate viewing angles may be selected from an array of viewing angles defined by discrete positions of the virtual X-ray source 210^{VS} on a virtual sphere surrounding the vascular region 130. The complete set of viewing angles in the array may be selected, or a subset of the viewing angles in the array may be selected based on considerations such as a feasibility criterion defining the achievability of the viewing angle taking into consideration factors such as a physician's access to the patient, a projection X-ray imaging system's ability to image a patient and so forth. The vessel overlap metric and/or vessel foreshortening metric may then be evaluated exhaustively for the viewing angles in the complete set/ subset. The minimum vessel overlap criterion and/or minimum vessel foreshortening criterion may then be applied by selecting the candidate viewing angle for which the corresponding virtual projection 130^{VP} provides a minimum value of the vessel overlap metric and/or vessel foreshortening metric.

Instead of evaluating the vessel overlap metric and/or vessel foreshortening metric exhaustively for the viewing angles in the complete set/ subset, an optimization procedure (e.g. Gradient Descent) may be performed wherein the array of viewing angles, or alternatively, viewing angles obtained from a continuously-variable angular space, optionally constrained by the feasibility criterion mentioned above, is explored iteratively in order to determine a candidate viewing angle for which the corresponding virtual projection 130^{VP} satisfies the minimum vessel overlap criterion and/or minimum vessel foreshortening criterion, for the least a portion of the vascular region 130. In this case, the candidate viewing angles for which the virtual projections, and the corresponding vessel overlap metric/ vessel foreshortening metric, is evaluated, are determined iteratively using the optimization procedure and based on the value of the metric evaluated for one or more earlier iterations.

In some examples, one or more further criteria may be used in addition to the minimum vessel overlap criterion and/or the minimum vessel foreshortening criterion, in order to determine the value of the viewing angle α₁, β₁ in the operation S120.

In one example, the one or more processors 110 are configured to receive input defining a point of interest in the vascular region 130. In this example, the one or more processors are configured to define the portion of the vascular region 130 for which the minimum vessel overlap criterion and/or the minimum vessel foreshortening criterion as a portion of one or more vessels within a predetermined distance of the point of interest. The one or more processors 110 are also configured to evaluate the minimum vessel overlap criterion and/or the minimum vessel foreshortening criterion, selectively within the portion of the vascular region 130.

By selectively evaluating the minimum vessel overlap criterion and/or the minimum vessel foreshortening criterion within the defined portion of the vascular region 130, the viewing angle is optimized for this portion of the vascular region. By way of an example, the viewing angle may be optimized so as to provide minimum value of the vessel overlap metric for a region of interest that is within a specified distance, e.g. 10 mm, around an LAD-D1 vessel bifurcation. The input may be received from a user, e.g. via a user input device such as a keyboard, or a mouse, or automatically, via e.g. the processor automatically detecting a point within a vessel (such as a point within an arterial stenosis) in the volumetric data.

In another example, at least a second value of a viewing angle is outputted by the system 100. In this example, the operation of determining S120 a value of a viewing angle α₁, β₁ for acquiring projection data 150 representing the vascular region 130, comprises:
identifying a first value of the viewing angle α₁, β₁ that meets the minimum vessel overlap criterion and/or the minimum vessel foreshortening criterion for the at least a portion of the vascular region 130; and
identifying at least a second value of the viewing angle, and wherein the at least a second value of the viewing angle satisfies a threshold criterion in relation to the minimum vessel overlap criterion and/or the minimum vessel foreshortening criterion; and
wherein the outputting S130 comprises outputting the first value of the viewing angle α₁, β₁; and
wherein the one or more processors 110 are configured to output the at least a second value of the viewing angle.

The at least a second value of the viewing angle provide alternative viewing angles for acquiring projection data. The at least a second value of the viewing angle may satisfy a threshold criterion such as meeting the minimum vessel overlap criterion and/or the minimum vessel foreshortening criterion within a specified tolerance. The at least a second value of the viewing angle may alternatively satisfy a threshold criterion such as providing the second-closest, third-closest, and so forth, match to the minimum vessel overlap criterion and/or the minimum vessel foreshortening criterion wherein the closest match is provided by the first value of the viewing angle α₁, β₁. The first and the at least a second values for the viewing angle may result from the exhaustive evaluation procedure, or the optimization procedure described above. The first and at least a second values may represent local minima in the optimization procedure. In one example, a difference between the first and the at least a second viewing angles exceeds a predetermined value. Thus, the local minima may be selected such that they represent significantly different orientations of a projection X-ray imaging system.

In another example, the value of the viewing angle α₁, β₁ satisfies the minimum vessel overlap criterion and/or the minimum vessel foreshortening criterion, for the least a portion of the vascular region 130, over at least a portion of the cardiac cycle, or over the at least a portion of the respiratory cycle, respectively.

As mentioned above, in some situations, a user may wish to determine the optimal viewing angle over a specified portion of the cardiac/ respiratory cycle, or over the complete cardiac/ respiratory cycle. For instance, a user may wish to determine the optimal viewing angle viewing a cardiovascular region for the duration of the diastole cardiac phase, or for the duration of the inhalation phase. In one example, the one or more processors 110 are configured to determine the value of the viewing angle α₁, β₁ based on the simulated motion 140 of the vascular region 130 and/or the motion of the vascular region 130 represented within the volumetric data 120, over at least a portion of a cardiac cycle, or over at least a portion of a respiratory cycle, and wherein the value of the viewing angle α₁, β₁ satisfies the minimum vessel overlap criterion and/or the minimum vessel foreshortening criterion, for the least a portion of the vascular region 130, over the at least a portion of the cardiac cycle, or over the at least a portion of the respiratory cycle, respectively.

This example provides an optimal viewing angle over the at least a portion of the cardiac/ respiratory cycle. In this example, the selection of the optimal viewing angle, or the optimization, is performed based on the values of the vessel overlap metric and/or the vessel foreshortening metric for the points in time over the at least a portion of the cardiac/ respiratory cycle. At each candidate viewing angle, a combined value of the vessel overlap metric and/or the vessel foreshortening metric is evaluated for the relevant points in time. The combined values of the vessel overlap metric and/or the vessel foreshortening metric for the different viewing angles are then used to determine the optimal viewing angle. In this example, the at least a portion of the cardiac/ respiratory cycle may be defined by user input, e.g. via a keyboard, or a mouse. Alternatively, the at least a portion of the cardiac/ respiratory cycle may be a default value provided by the one or more processors of the system.

In another example, a projection X-ray imaging system viewing angle feasibility criterion is used to determine the value of the viewing angle α₁, β₁ in the operation S 120. The feasibility criterion may be used in addition to the minimum vessel overlap criterion and/or the minimum vessel foreshortening criterion. A projection X-ray imaging system viewing angle feasibility criterion defines a range of viewing angles that are achievable by a projection X-ray imaging system based on factors such as the extent to which objects, such as an element of the projection X-ray imaging system, the patient bed, and the anatomy of the patient, hamper a physician's access to a patient, or hamper a projection X-ray imaging system's ability to image a patient. A projection X-ray imaging system viewing angle feasibility criterion may exclude specific (ranges of) angle(s) at which a C-arm, or an X-ray source, or an X-ray detector, hamper a physician's access to a patient, for example. Similarly, a projection X-ray imaging system viewing angle feasibility criterion may exclude specific (ranges of) angle(s) at which a patient's arm hampers the projection X-ray imaging system's access to a patient. The projection X-ray imaging system viewing angle feasibility criterion may be used to determine the value of the viewing angle α₁, β₁ in the operation S120 by constraining the candidate viewing angles for which the vessel overlap metric/ vessel foreshortening metric is evaluated using the feasibility criterion.

Taking now the second set of examples in which the value of the viewing angle α₁, β₁ is determined using the volumetric data 120, and based on a motion of the vascular region 130 represented within the volumetric data 120. In these examples, the volumetric data inherently represents the motion of the vascular region via changes in the shape and/or position of vessel(s) in the vascular region 130 over time. In these examples, the volumetric data includes a temporal sequence of volumetric images representing the vascular region 130. The volumetric images may represent the vascular region at a plurality of corresponding points in the cardiac cycle, or at a plurality of corresponding points in the respiratory cycle, for example.

In the second set of examples, the one or more processors 110 are configured to determine the value of the viewing angle α₁, β₁ based on the motion of the vascular region 130 represented within the volumetric data 120, and the determining the value of the viewing angle α₁, β₁ comprises:
analyzing the volumetric data 120 to determine a volumetric shape of the vascular region 130 at each of one or more points in time;
for each of a plurality of candidate viewing angles α_{i⊂1..m}, β_{j⊂1..m}:
evaluating a vessel overlap metric and/or a vessel foreshortening metric for at least a portion of the volumetric shape of the vascular region 130 for the one or more points in time; and
determining the value of the viewing angle α₁, β₁ based on the vessel overlap metrics and/or the vessel foreshortening metrics corresponding to the one or more points in time for the candidate viewing angles α_{i⊂1..m}, β_{j⊂1..m}.

In the second set of examples, the operation of analyzing the volumetric data 120 to determine a volumetric shape of the vascular region 130 at a point in time, may be performed in the manner described above for the first set of examples.

In the second set of examples, the operation of evaluating the vessel overlap metric and the vessel foreshortening metric may be performed in the manner described above.

In one example, the operation of evaluating a vessel overlap metric and/or a vessel foreshortening metric for at least a portion of the volumetric shape of the vascular region 130 for the one or more points in time, comprises:
projecting the volumetric shape of the vascular region 130 for the one or more points in time, from the candidate viewing angle α_{i⊂1..m}, β_{j⊂1..m}, to provide a virtual projection of the volumetric shape of the vascular region 130 for the one or more points in time; and
evaluating the vessel overlap metric and/or the vessel foreshortening metric for the at least a portion of the vascular region 130 for the one or more points in time based on the virtual projections of the volumetric shapes of the vascular region 130.

The virtual projections may be generated in the manner described above for the first set of examples with reference to Fig. 3 - Fig. 5.

In the second set of examples, the value of the viewing angle α₁, β₁ is then determined based on the virtual projections of the volumetric shapes of the vascular region 130 corresponding to the one or more points in time for each candidate viewing angle α_{i⊂1..m}, β_{j⊂1..m}. The operation of determining the value of the viewing angle α₁, β₁ may be performed based on a minimum vessel overlap criterion and/or a minimum vessel foreshortening criterion for at least a portion of the vascular region 130. The minimum vessel overlap criterion and/or the minimum vessel foreshortening criterion is evaluated based on a calculated value of a respective vessel overlap metric and/or a vessel foreshortening metric for the at least a portion of the vascular region 130 at each of a plurality of candidate viewing angles α_{i⊂1..m}, β_{j⊂1..m}. These criteria may be evaluated in the manner described above for the first set of examples. For instance, in one example, the one or more processors 110 are configured to evaluate the minimum vessel overlap criterion and/or the minimum vessel foreshortening criterion, based on a virtual projection 130^{VP} of the vascular region 130 from each of the plurality of candidate viewing angles α_{i⊂1..m}, β_{j⊂1..m}.

In one example, the operation of determining the value of the viewing angle α₁, β₁ comprises interpolating a volumetric shape of the vascular region 130 to provide a volumetric shape of the vascular region at one or more intermediate points in time. The operation of determining the value of the viewing angle α₁, β₁, is performed using the vessel overlap metric and/or the vessel foreshortening metric evaluated for the one or more intermediate points in time. A more accurate viewing angle may be provided by determining the viewing angle using the shapes of the vascular region 130 corresponding to the one or more intermediate points in time, due to the increased temporal granularity provided by the intermediate points in time.

In one example, , the one or more processors 110 are configured to:
analyze the volumetric data 120 to provide a vascular model representing a volumetric shape of the vascular region 130 and/or an anatomical model representing an anatomical region coupled to the vascular region 130. The motion of the vascular region 130 represented within the volumetric data 120, is determined using the vascular model and/or the anatomical model, respectively.

In this example, the operation of analyzing of the volumetric data 120 to provide the vascular model may be preformed using various known image segmentation techniques, neural networks, and path-finding algorithms, as described above for the first set of examples. Instead of, or in addition to, providing the vascular model, the volumetric data 120 may be analyzed in order to provide an anatomical model representing an anatomical region coupled to the vascular region 130. The anatomical model may represent a portion of the anatomy such as a portion of cardiac surface, or a portion of a lung, and which is coupled to the vascular region. The anatomical model may provide useful information relating to the motion of the vascular region. For instance, an anatomical model representing a cardiac surface, may be used to extract motion data that is useful in determining the motion of the vascular region 130. The anatomical model may be obtained from the volumetric data using the analysis techniques described above, for example via segmentation, or via the use of a trained neural network.

Referring now to the operation S 130 mentioned above with reference to Fig. 1 and Fig. 2; in this operation, the value of the viewing angle is outputted. This operation is performed for both the first set of examples, and also for the second set of examples. The value of the viewing angle may be outputted in various ways. For example, the value of the viewing angle may be outputted to a display device, such as to the display 220 illustrated in Fig. 1, or to a virtual/ augmented reality display device, or to a printer, or to a computer-readable storage medium, or to the Internet, or to the Cloud, and so forth.

In a related example, the one or more processors are configured to output a measure of vessel overlap and/or a measure of vessel foreshortening for the vascular region 130. The measures of vessel overlap, and vessel foreshortening, may be evaluated using the vessel overlap metric, and the vessel foreshortening metric described above. The measures of vessel overlap, and vessel foreshortening, may be outputted in a similar manner to the value of the viewing angle.

In another example, the value of the viewing angle that is determined by the system 100 is outputted to a projection X-ray imaging system. In this example, the one or more processors 110 are configured to:
cause the projection X-ray imaging system to adjust a current viewing angle of the projection X-ray imaging system to the value of the viewing angle; and
cause the projection X-ray imaging system to acquire projection X-ray data at the value of the viewing angle.

This reduces the risk of acquiring projection images that give a misleading impression of the vasculature. In this example, the operations of adjusting a current viewing angle of the projection X-ray imaging system, and causing the projection X-ray imaging system to acquire projection X-ray data, may be performed subject to user confirmation of prompts outputted by the one or more processors. For example, the one or more processors may output a prompt to a display device indicating a suggestion to adjust the current viewing angle. Subsequent to a user's confirmation of the prompt, the one or more processors may cause the projection X-ray imaging system to adjust the current viewing angle of the projection X-ray imaging system to the value of the viewing angle outputted by the one or more processors. Similarly, the one or more processors may output a prompt to a display device indicating a suggestion to acquire projection X-ray data. Subsequent to a user's confirmation of the prompt, the one or more processors may cause the projection X-ray imaging system to acquire projection X-ray data at the value of the viewing angle. This ensures that the user remains in control of safety aspects associated with adjusting the viewing angle, and acquiring acquire projection X-ray data, respectively.

In another example, the one or more processors 110 are configured to output a graphical representation 170 of the vascular region 130. The graphical representation includes, for the outputted viewing angle α₁, β₁, an indication of an extent of the motion of the vascular region 130 over the at least a portion of the cardiac cycle, or over the at least a portion of the respiratory cycle, respectively.

In this example, the extent of the motion of the vascular region 130 may be evaluated for the portion of the cardiac cycle using the vessel foreshortening metric described above. In one example, the extent of the motion of the vascular region 130 is indicated as a static value. The static value may represent a maximum or an average value of the vessel foreshortening metric, or an amount of variation in the vessel foreshortening metric, for the at least a portion of the cardiac/ respiratory cycle. In another example, the extent of the motion of the vascular region 130 is indicated dynamically. For example, the graphical representation 170 of the vascular region 130 may dynamically display changes in the amount of foreshortening over the at least a portion of the cardiac/ respiratory cycle. In this example, the changes in the amount of foreshortening may be evaluated using the vessel foreshortening metric described above. For example, a graphical representation may be provided in a similar manner to that illustrated in Fig. 7 - Fig. 9, and wherein the extent of the motion of the vascular region 130 is indicated dynamically by showing the vessel foreshortening as a graph, or as a color-coded overlay on the vessel(s) in the vascular region, for each of a plurality of points in time over the at least a portion of the cardiac/ respiratory cycle.

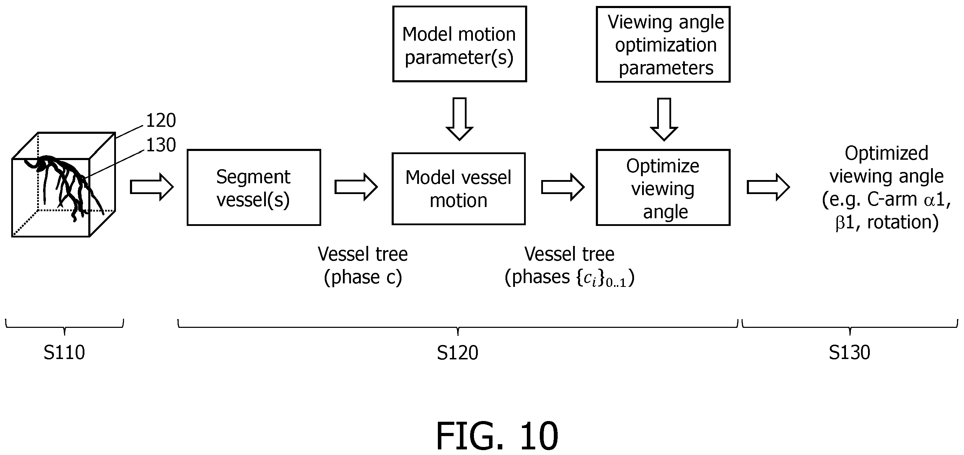

Fig. 10 is a schematic diagram illustrating an example of various aspects of a method of providing a viewing angle α₁, β₁, for acquiring projection data of a vascular region, in accordance with some aspects of the present disclosure. Fig. 10 provides an example implementation of some of the various operations described above. Starting on the left-hand side of Fig. 10, the receiving of the volumetric data 120 in the operation S110, is illustrated. In this example, the volumetric data 120 comprises a single static image of the vascular region 130 at a specific cardiac phase, c. The cardiac phase, c, may be determined by performing an image processing operation on the volumetric data as described above, or it may be obtained from ECG data that is acquired contemporaneously with the volumetric data 120. Moving towards the right-hand side of Fig. 10, various elements of the operation S120 are illustrated. In this example, the volumetric data 120 is segmented in order to provide a vessel tree, such as a cardiac vessel tree for example. In the illustrated example, the volumetric data 120 represents the vessel tree in the specific cardiac phase, c. Moving towards the right-hand side of Fig. 10, the vessel motion is then modelled for the vessels in the vessel tree, e.g. using the parametric motion model described above, in order to provide a vessel tree at each of multiple further cardiac phases *cᵢ*. The multiple further cardiac phases *cᵢ* may represent the complete cardiac cycle, or a portion thereof. In order to determine the vessel tree for the multiple cardiac phases *cᵢ*, the parametric motion model is inputted with various motion parameters. These may define factors such as the cardiac phase, c, the cardiac phase interval, the heart rate, the size of the vessel tree, and so forth. Moving towards the right-hand side of Fig. 10, the optimal value of the viewing angle is then determined. This operation may be performed by projecting the vessel tree for each of the cardiac phases *cᵢ*, from an initial viewing angle, evaluating a vessel overlap metric and/or a vessel foreshortening metric at the initial viewing angle, and using an optimization procedure (e.g. Gradient Descent) to determine subsequent viewing angles at which to evaluate the evaluate the metric(s). The optimization procedure ends when a stopping criterion is met, e.g. a specified number of iterations has been performed, or a specified time has elapsed, or when the value of the metric reaches a threshold value. The optimized value of the viewing angle is then outputted, e.g. to a display, or to a projection X-ray imaging system. A projection X-ray image of the vascular region 130 may then be acquired using the outputted value of the viewing angle.

It is noted that the system 100 may also include one or more of: a volumetric imaging system for providing the volumetric data, such as for example the a CT imaging system, an MRI imaging system, a 3D ultrasound imaging system, or a 3DRA system; a projection imaging system for acquiring projection data of the vascular region, such as the projection X-ray imaging system 210 illustrated in Fig. 1; a display, such as the display 220 illustrated in Fig. 1, for displaying the value of the viewing angle determined by the system, the volumetric data, the projection data, other output data generated by the one or more processors 110, and so forth; a patient bed 230; and a user input device (not illustrated in Fig. 1) configured to receive user input in relation to the operations performed by the one or more processors 110, such as a keyboard, a mouse, a touchscreen, and so forth.

In another example, a computer-implemented method of providing a viewing angle for acquiring projection data of a vascular region, is provided. The method comprises:
receiving S110 volumetric data 120 representing the vascular region 130;
determining S120, using the volumetric data 120, and based on a simulated motion 140 of the vascular region 130 and/or based on a motion of the vascular region 130 represented within the volumetric data 120, a value of a viewing angle α₁, β₁ for acquiring projection data 150 representing the vascular region 130; and
outputting S130 the value of the viewing angle α₁, β₁.

In another example, a computer program product, is provided. The computer program product comprises instructions which when executed by one or more processors, cause the one or more processors to carry out a method of providing a viewing angle for acquiring projection data of a vascular region. The method comprises:
receiving S110 volumetric data 120 representing the vascular region 130;
determining S120, using the volumetric data 120, and based on a simulated motion 140 of the vascular region 130 and/or based on a motion of the vascular region 130 represented within the volumetric data 120, a value of a viewing angle α₁, β₁ for acquiring projection data 150 representing the vascular region 130; and
outputting S130 the value of the viewing angle α₁, β₁.

In another example, a projection X-ray imaging system 210 is provided. The projection X-ray imaging system comprises one or more processors 110 configured to:
receive S 110 volumetric data 120 representing the vascular region 130;
determine S 120, using the volumetric data 120, and based on a simulated motion 140 of the vascular region 130 and/or based on a motion of the vascular region 130 represented within the volumetric data 120, a value of a viewing angle α₁, β₁ for acquiring projection data 150 representing the vascular region 130; and
output S130 the value of the viewing angle α₁, β₁; and
wherein the one or more processors 110 are configured to:
   cause the projection X-ray imaging system 210 to adjust a current viewing angle of the projection X-ray imaging system to the value of the viewing angle α₁, β₁; and
   cause the projection X-ray imaging system to acquire projection X-ray data 150 representing the vascular region 130 at the value of the viewing angle α₁, β₁.

In this example, the operations of adjusting a current viewing angle of the projection X-ray imaging system, and causing the projection X-ray imaging system to acquire projection X-ray data, may be performed subject to user confirmation of prompts outputted by the one or more processors, as described above.

The above examples are to be understood as illustrative of the present disclosure, and not restrictive. Further examples are also contemplated. For instance, the examples described in relation to a computer-implemented method, may also be provided by the computer program product, or by the computer-readable storage medium, or by the system 100, or by the X-ray projection imaging system that includes the system 100, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A system (100) for providing a viewing angle for acquiring projection data of a vascular region, the system comprising one or more processors (110) configured to:
receive (S110) volumetric data (120) representing the vascular region (130);
determine (S120), using the volumetric data (120), and based on a simulated motion (140) of the vascular region (130) and/or based on a motion of the vascular region (130) represented within the volumetric data (120), a value of a viewing angle (α₁, β₁) for acquiring projection data (150) representing the vascular region (130); and
output (S130) the value of the viewing angle (α₁, β₁).

2. The system according to claim 1, wherein the one or more processors (110) are configured to determine the value of the viewing angle (α₁, β₁) based on a minimum vessel overlap criterion and/or a minimum vessel foreshortening criterion for at least a portion of the vascular region (130); and
wherein the minimum vessel overlap criterion and/or the minimum vessel foreshortening criterion is evaluated based on a calculated value of a respective vessel overlap metric and/or a vessel foreshortening metric for the at least a portion of the vascular region (130) at each of a plurality of candidate viewing angles (α_{i⊂1..m}, β_{j⊂1..m}).

3. The system according to claim 2, wherein the one or more processors (110) are configured to evaluate the minimum vessel overlap criterion and/or the minimum vessel foreshortening criterion, based on a virtual projection (130^{VP}) of the vascular region (130) from each of the plurality of candidate viewing angles (α_{i⊂1..m}, β_{j⊂1..m}).

4. The system according to claim 2 or claim 3, wherein the one or more processors (110) are further configured to receive input defining a point of interest in the vascular region (130), and to define the portion of the vascular region (130) as a portion of one or more vessels within a predetermined distance of the point of interest; and wherein the one or more processors (110) are configured to evaluate the minimum vessel overlap criterion and/or the minimum vessel foreshortening criterion, selectively within the portion of the vascular region (130).

5. The system according to any one of claims 2-4, wherein the determining (S120) comprises:
identifying a first value of the viewing angle (α₁, β₁) that meets the minimum vessel overlap criterion and/or the minimum vessel foreshortening criterion for the at least a portion of the vascular region (130); and
identifying at least a second value of the viewing angle, and wherein the at least a second value of the viewing angle satisfies a threshold criterion in relation to the minimum vessel overlap criterion and/or the minimum vessel foreshortening criterion; and
wherein the outputting (S130) comprises outputting the first value of the viewing angle (α₁, β₁); and
wherein the one or more processors (110) are further configured to output the at least a second value of the viewing angle.

6. The system according to any one of claims 2-5, wherein the one or more processors (110) are configured to determine the value of the viewing angle (α₁, β₁) based on the simulated motion (140) of the vascular region (130) and/or the motion of the vascular region (130) represented within the volumetric data (120), over at least a portion of a cardiac cycle, or over at least a portion of a respiratory cycle, and wherein the value of the viewing angle (α₁, β₁) satisfies the minimum vessel overlap criterion and/or the minimum vessel foreshortening criterion, for the least a portion of the vascular region (130), over the at least a portion of the cardiac cycle, or over the at least a portion of the respiratory cycle, respectively.

7. The system according to claim 6, wherein the one or more processors (110) are further configured to output a graphical representation (170) of the vascular region (130); and
wherein the graphical representation includes, for the outputted viewing angle (α₁, β₁), an indication of an extent of the motion of the vascular region (130) over the at least a portion of the cardiac cycle, or over the at least a portion of the respiratory cycle, respectively.

8. The system according to any previous claim, wherein the one or more processors (110) are further configured to:
simulate the motion of the vascular region (130) using a parametric motion model, to provide the simulated motion (140) of the vascular region (130);
and/or
analyze the volumetric data (120) to determine the motion of the vascular region (130), respectively.

9. The system according to claim 8, wherein the vascular region is a cardiovascular region, and wherein the parametric motion model represents the motion of the heart over at least a portion of a cardiac cycle.

10. The system according to claim 8 or claim 9, wherein the parametric motion model comprises one or more patient parameters; and
wherein the one or more processors (110) are configured to simulate the motion (140) of the vascular region (130) using the patient parameters.

11. The system according to claim 8, wherein the one or more processors (110) are further configured to:
analyze the volumetric data (120) to determine a motion state of the vascular region (130) and/or to receive sensor data representing a motion state of the vascular region (130); and
wherein the one or more processors (110) are configured to determine the value of the viewing angle (α₁, β₁) based further on the motion state.

12. The system according to any previous claim, wherein the one or more processors (110) are further configured to:
analyze the volumetric data (120) to provide a vascular model representing a volumetric shape of the vascular region (130) and/or an anatomical model representing an anatomical region coupled to the vascular region (130); and
wherein the simulated motion (140) of the vascular region (130) and/or the motion of the vascular region (130) represented within the volumetric data (120), is determined using the vascular model and/or the anatomical model, respectively.

13. The system according to any previous claim, wherein
i) the one or more processors (110) are configured to determine the value of the viewing angle (α₁, β₁) based on a simulated motion (140) of the vascular region (130), and wherein the determining the value of the viewing angle (α₁, β₁) comprises:
analyzing the volumetric data (120) to determine a volumetric shape of the vascular region (130) at a point in time;
determining, based on a simulated motion (140) of the vascular region (130), a simulated volumetric shape of the vascular region (130) at each of one or more different points in time; and
for each of a plurality of candidate viewing angles (α_{i⊂1..m}, β_{j⊂1..m}):
evaluating a vessel overlap metric and/or a vessel foreshortening metric for at least a portion of the simulated volumetric shape of the vascular region (130) for the one or more different points in time; and
determining the value of the viewing angle (α₁, β₁) based on the vessel overlap metrics and/or the vessel foreshortening metrics corresponding to the one or more different points in time for the candidate viewing angles (α_{i⊂1..m}, β_{j⊂1..m});
or
ii) the one or more processors (110) are configured to determine the value of the viewing angle (α₁, β₁) based on the motion of the vascular region (130) represented within the volumetric data (120), and wherein the determining the value of the viewing angle (α₁, β₁) comprises:
analyzing the volumetric data (120) to determine a volumetric shape of the vascular region (130) at each of one or more points in time;
for each of a plurality of candidate viewing angles (α_{i⊂1..m}, β_{j⊂1..m}):
evaluating a vessel overlap metric and/or a vessel foreshortening metric for at least a portion of the volumetric shape of the vascular region (130) for the one or more points in time; and
determining the value of the viewing angle (α₁, β₁) based on the vessel overlap metrics and/or the vessel foreshortening metrics corresponding to the one or more points in time for the candidate viewing angles (α_{i⊂1..m}, β_{j⊂1..m}).

14. The system according to claim 13, wherein
i) the evaluating a vessel overlap metric and/or a vessel foreshortening metric for at least a portion of the simulated volumetric shape of the vascular region (130) for the one or more different points in time, comprises:
projecting the simulated volumetric shape of the vascular region (130) for the one or more different points in time, from the candidate viewing angle (α_{i⊂1..m}, β_{j⊂1..m}), to provide a virtual projection of the simulated volumetric shape of the vascular region (130) for the one or more different points in time; and
evaluating the vessel overlap metric and/or the vessel foreshortening metric for the at least a portion of the vascular region (130) for the one or more points in time based on the virtual projections of the simulated volumetric shapes of the vascular region (130);
or
ii) the evaluating a vessel overlap metric and/or a vessel foreshortening metric for at least a portion of the volumetric shape of the vascular region (130) for the one or more points in time, comprises:
projecting the volumetric shape of the vascular region (130) for the one or more points in time, from the candidate viewing angle (α_{i⊂1..m}, β_{j⊂1..m}), to provide a virtual projection of the volumetric shape of the vascular region (130) for the one or more points in time; and
evaluating the vessel overlap metric and/or the vessel foreshortening metric for the at least a portion of the vascular region (130) for the one or more points in time based on the virtual projections of the volumetric shapes of the vascular region (130).

15. The system according to claim 13, wherein the determining the value of the viewing angle (α₁, β₁) further comprises interpolating a volumetric shape of the vascular region (130) to provide a volumetric shape of the vascular region at one or more intermediate points in time; and
wherein the determining the value of the viewing angle (α₁, β₁), is performed using the vessel overlap metric and/or the vessel foreshortening metric evaluated for the one or more intermediate points in time.
